# FASCICULE DE BREVET EUROPEEN

(11) **EP 3 359 587 B1**
(45) Date de publication et mention de la délivrance du brevet: **29.06.2022**
(21) Numéro de dépôt: 16775503.2
(22) Date de dépôt: 16.09.2016
(51) Int. Cl.: C08H 99/00, C08L 99/00, C08L 3/02, C08L 5/00

(54) **PROCÉDÉ DE PRÉPARATION D'UNE POUDRE D'ALGUES À TENEUR RÉDUITE EN PROTÉINES ET COMPOSITION BIOPLASTIQUE FORMULÉE À PARTIR D'UNE TELLE POUDRE**
VERFAHREN ZUR HERSTELLUNG EINES ALGENPULVERS MIT REDUZIERTEM PROTEINGEHALT UND AUS SOLCH EINEM PULVER FORMULIERTE BIOKUNSTSTOFFZUSAMMENSETZUNG
METHOD FOR PREPARING AN ALGAE POWDER WITH REDUCED PROTEIN CONTENT AND BIOPLASTIC COMPOSITION FORMULATED FROM SUCH A POWDER

(30) Priorité: 17.09.2015 FR 1558743
(43) Date de publication de la demande: 15.08.2018
(73) Titulaire: Eranova, 73370 Le Bourget du Lac (FR)
(72) Inventeur: LAVOISIER, Philippe, 73370 Le Bourget du Lac (FR); PIERRE, Ronan, 22610 Pleubian (FR); BENOIT, Maud, 22610 Pleubian (FR)
(74) Mandataire: Cabinet Le Guen Maillet
(86) Numéro de dépôt international: PCT/EP2016/072020
(87) Numéro de publication internationale: WO 2017/046356

(56) Documents cités:
- WO-A1-2012/114045
- WO-A1-2014/128411
- WO-A2-2010/125490
- FR-A1- 3 012 817
- US-A- 5 779 960
- "Sustainable algal biomass products by cultivation in waste water flows", , 2013, XP055234319, Extrait de l'Internet: URL:http://www.vtt.fi/inf/pdf/technology/2 013/T147.pdf [extrait le 2015-12-07]
- CHIELLINI E ET AL: "Biodegradable Thermoplastic Composites Based on Polyvinyl Alcohol and Algae", BIOMACROMOLECULES, AMERICAN CHEMICAL SOCIETY, US, vol. 9, 8 février 2008 (2008-02-08), pages 1007-1013, XP002491632, ISSN: 1525-7797, DOI: 10.1021/BM701041E [extrait le 2008-02-08]

## Description

La présente invention appartient au domaine technique des matériaux formulés à partir de matières naturelles, plus précisément des matériaux plastiques, et concerne un procédé de préparation d'une poudre d'algues, à teneur réduite en protéines, une composition bioplastique formulée à partir d'une telle poudre, un procédé de fabrication d'un produit plastique obtenu à partir d'une telle poudre d'algues ainsi que le produit plastique ainsi obtenu.

Un produit plastique est un produit obtenu à partir d'une composition contenant des polymères auxquels sont ajoutés différents additifs, tels que des plastifiants. Le plastique traditionnel est composé de polymères issus de l'industrie pétrolière. Mais l'épuisement des ressources pétrolières et la préservation de l'environnement ont motivé la recherche de composés non issus du pétrole et les plastiques formulés à base de composés d'origine naturelle sont une alternative intéressante aux plastiques traditionnels.

Les produits en matière plastique sont souvent fabriqués par extrusion. Le matériau de départ qui se trouve sous forme de granulés ou de poudre est passé dans une extrudeuse laquelle permet de produire en continu des pièces telles que des profilés, câbles, tubes, feuilles, films, fibres, plaques, joncs, etc.

Les algues sont aujourd'hui considérées comme des sources de polymères valorisables. Ceux-ci ont prouvé leur efficacité dans des domaines variés tels que les domaines de l'alimentaire, de la pharmacie, du carburant ou de la cosmétique. En outre, la prolifération des algues le long des côtes marines devient un phénomène inquiétant et de grande ampleur.

En ce qui concerne le domaine des produits plastiques, les algues représentent une source de matières premières intéressantes du fait de leur richesse en polymères.

Les compositions bioplastiques réalisées à partir de macro algues ou de micro algues sont généralement produites par broyage des matières algales séchées, puis mélange avec d'autres composants tels que des plastifiants. On citera à ce titre la demande de brevet publiée US 2013/0220173.

Les compositions bioplastiques à base d'algues sont ensuite généralement utilisées sous forme de granulés, après passage dans une extrudeuse à vis.

Les produits plastiques pouvant être fabriqués à partir de ces granulés sont des produits injectés, thermoformés, extrudés en bulles, extrudés en gonflage.

Les micro algues ou macro algues utilisées pour la préparation des compositions bioplastiques sont généralement des algues vertes ou brunes. Il est intéressant de pouvoir les utiliser dans leur intégralité, c'est à dire avec tous les composants de l'algue: protéines, polysaccharides, minéraux, etc....en effet, on connaît de nombreuses compositions bioplastiques qui sont formulées à partir de polysaccharides d'algues extraits, purifiés et concentrés. Par exemple, de nombreux produits plastiques comportent des alginates en tant que composants. De tels procédés sont coûteux, longs et nécessitent des étapes d'extractions fines.

Les compositions élaborées à partir d'algues entières réduites en poudre confèrent des propriétés mécaniques réduites aux produits plastiques formés. Ceux-ci sont relativement cassants. En outre, les poudres d'algues sont utilisées comme charges généralement à hauteur d'environ 15% en poids, au maximum. Dans les filières de valorisation des algues, il serait avantageux de pouvoir augmenter le taux d'algues dans les produits plastiques.

Un autre inconvénient majeur des compositions bioplastiques à base de micro algues ou macro algues est qu'elles présentent une forte odeur et une couleur, par exemple brune, très prononcée. On note, en outre, que les problèmes d'odeur et de couleur s'accentuent lors de la fabrication des produits plastiques, en particulier lors des procédés d'extrusion, de moulage, d'injection, de thermo compression, etc., des compositions bioplastiques.

Pour pallier ce problème, certains procédés incluent l'ajout de composés aptes à absorber ou à masquer l'odeur, tels que du charbon actif, et de colorants. Il est nécessaire dans ce cas de procéder à un travail de sélection tant de l'origine que de la quantité du composant à ajouter afin d'obtenir des mélanges stables, efficaces, naturels et d'éviter toute incompatibilité entre composants. Les résultats obtenus ne sont pas toujours satisfaisants et le travail de sélection est une contrainte supplémentaire.

De récents travaux de recherche ont permis d'obtenir des avancées techniques permettant d'améliorer les compositions bioplastiques formulées à partir de composants d'algues, notamment d'augmenter la teneur en matière algale dans ces compositions et d'obtenir des produits plastiques présentant de bonnes propriétés mécaniques.

A ce titre, la demande de brevet Français FR 3 012 817 décrit des compositions bioplastiques formulées à partir d'un composant d'algues, de plastifiants d'origine naturelle, tels que du glycérol, et de polymères d'origine végétale, tels que l'amidon. De manière préférée, le composant d'algues se présente sous la forme d'un résidu d'algues contenant des polysaccharides qualifiés de semi-raffinés, car non totalement extraits et non purifiés. Il a été observé une meilleure plastification de l'amidon dans les compositions de bioplastiques réalisées à partir des résidus contenant des polysaccharides semi-raffinés, par rapport à des extraits raffinés, donc purifiés, des mêmes polysaccharides.

Par ailleurs, il a été démontré dans la demande de brevet français FR 3 012 817 qu'en utilisant des conditions de culture spécifiques de la biomasse algale, il était possible d'augmenter la synthèse endogène d'amidon. Il a en outre été démontré que l'utilisation d'une biomasse algale chez laquelle la synthèse endogène d'amidon avait été accentuée avant la préparation d'un résidu d'algues contenant des polysaccharides semi-raffinés, permettait d'obtenir des produits plastiques aux propriétés mécaniques avantageuses, notamment de bonnes résistances aux cassures. Les propriétés mécaniques de ces bioplastiques réalisés du fait de la richesse en amidon sont supérieures à celles des bioplastiques obtenus par simple séchage et broyage des micro ou macro algues.

Cependant, les compositions bioplastiques et produits bioplastiques décrits dans la demande de brevet susmentionnée, bien que présentant de bonnes caractéristiques mécaniques, présentent eux aussi de mauvaises caractéristiques d'odeur et une couleur foncée ou brune persistante au contraire de la transparence ou de la couleur claire recherchée dans les produits plastiques.

C'est dans ce contexte que la société déposante a mené des recherches afin de remédier aux problèmes de couleur et d'odeur des compositions bioplastiques et produits bioplastiques obtenus à partir d'algues.

Les macro algues et les micro algues sont composées essentiellement de glucides, de protéines, de pigments et de minéraux. En fonction des espèces et de leur période de récolte, les teneurs en ces composants peuvent largement varier. Par exemple selon la saison, les algues produisent davantage de protéines qui constituent leurs réserves et qui seront utilisées durant leur période de croissance.

Lors de la formation des bioplastiques, les matières sont soumises à des températures élevées et à des taux de cisaillement importants. A de telles températures, les acides aminés des protéines réagissent avec les polysaccharides suivant une réaction dite réaction de Maillard. La réaction de Maillard est une réaction connue qui a fait et qui fait l'objet de nombreuses études afin de tenter d'en identifier tous les mécanismes. De nombreux ouvrages en décrivent de manière détaillée les principes qui, de ce fait, ne seront pas exposés dans la présente description.

La société déposante a mis en évidence que les réactions de Maillard étaient également responsables des phénomènes de coloration et de dégagement d'odeurs indésirables durant les procédés de fabrication des bioplastiques, du fait de la synthèse de composés aromatiques et odorants. Elle a donc travaillé de manière à résoudre ce problème technique.

La présente invention a pour but de pallier les inconvénients susmentionnés et, en particulier, de proposer un procédé permettant l'obtention d'une poudre d'algues apte à être utilisée dans la préparation de compositions bioplastiques et de produits bioplastiques dépourvus d'odeur et de couleur indésirables.

Un autre but de l'invention est de proposer une telle poudre d'algues qui soit également apte à être utilisée dans la préparation de compositions plastiques présentant des caractéristiques de plastifications optimum et dans la préparation de produits plastiques présentant des propriétés mécaniques optimum, telles que la résistance à la cassure, traction, à la tension et l'allongement à la rupture.

Enfin, un autre but de l'invention est de proposer une composition de bioplastique et des produits bioplastiques élaborés à partir d'une poudre d'algues selon l'invention.

A cet effet, l'invention concerne un procédé de préparation d'une poudre d'algues, notamment destinée à la fabrication d'un produit plastique, comprenant les étapes successives de :
- culture et/ou récolte d'une biomasse d'algues,
- réduction d'au moins 7 0 % de la quantité intrinsèque en protéines des algues, en masse par rapport à la masse de protéines de la biomasse récoltée,
- séchage,
- réduction en poudre ou granulés .

La réduction de la teneur en protéines intrinsèque dans les cellules composant la biomasse algale permet de réduire les phénomènes de réactions de Maillard et permet l'obtention d'une poudre apte à être utilisée pour la préparation de compositions et de produits bioplastiques présentant de bonnes caractéristiques en termes de réduction, voire d'absence d'odeurs et de colorations indésirables.

L'étape de réduction est une étape de réduction d'au moins 70 %, préférentiellement d'au moins 75% de la quantité intrinsèque en protéines des algues, en masse par rapport à la masse de protéines de la biomasse récoltée.

Le procédé selon l'invention permet en effet de réduire jusqu'à plus de 75 % la quantité de protéines initialement présente dans la biomasse d'algues. Par là même, il permet d'obtenir une poudre d'algue présentant un taux de protéines, par rapport à la masse totale de la poudre, très réduit.

Selon un mode de réalisation préféré de l'invention, la réduction de la quantité de protéines intrinsèque des algues récoltées comprend :
- l'hydrolyse enzymatique des protéines intrinsèques par mélange de la biomasse algale récoltée, préférentiellement broyée, et d'une ou plusieurs protéases, puis,
- la séparation d'un hydrolysat enrichi en matière protéique et d'un résidu d'algues.

Le procédé selon le mode de réalisation préféré de l'invention consiste à réaliser une hydrolyse enzymatique des protéines des algues et la séparation de la matière protéique obtenue, riche en peptides et acides aminés, d'un résidu d'algues dans lequel le taux de protéines est significativement diminué. Les compositions bioplastiques réalisées à partir de ce résidu d'algues sont non ou peu colorées et dépourvues d'odeur. Les produits extrudés et injectés par transformation des granulés de bioplastiques sont également dépourvus d'odeur et de couleur. De manière surprenante, ces produits possèdent des propriétés mécaniques supérieures aux produits plastiques obtenus à partir de poudres d'algues séchées ou de poudres d'algues préparées selon un procédé décrit dans la demande de brevet français FR 3 012 817.

Selon un tel procédé d'hydrolyse enzymatique, la quantité de protéines des algues peut être réduite d'au moins 40 %, préférentiellement entre 40 et 80 %, plus préférentiellement entre 50 et 75%, en poids par rapport au poids total initial des protéines.

Par ailleurs, les hydrolysats de matière protéique récupérés présentent une valeur économique importante pour des applications telles que des applications de cosmétiques, d'engrais, de nutrition, notamment de nutrition animale. Cette valorisation de sous produits à haute valeur ajoutée du procédé objet de l'invention, résultant de ce traitement enzymatique, renforce le modèle économique de la culture algale.

Avantageusement, l'hydrolyse enzymatique des protéines est réalisée à l'aide d'une ou plusieurs protéases, en particulier d'une ou plusieurs endopeptidases et/ou exopeptidases, d'origine bactérienne ou fongique, telles qu'une ou plusieurs enzymes obtenues à partir de *Bacillus licheniformis, Bacillus subtilis, Bacillus amyloliquefaciens* ou *Aspergillus oryzae,* préférentiellement, de *Bacillus licheniformis.*

En fonction de la nature de l'enzyme utilisée et, ou, de la nature de l'algue traitée, le taux de réduction de la quantité de protéines va varier. Les enzymes d'origine bactérienne ou fongique utilisées dans le cadre de la présente invention sont toutes disponibles dans le commerce. Par exemple, à titre d'enzyme obtenue à partir de *Bacillus licheniformis,* on citera l'Alcalase commercialisée par la société Novozymes. A titre d'enzyme d'origine fongique obtenue à partir *d'Aspergillus oryzae,* on citera Flavourzyme commercialisée également par la société Novozymes. Généralement, l'enzyme est ajoutée à une concentration comprise entre 0,1 et 10 % par rapport à la masse de protéines à hydrolyser, préférentiellement entre 0,8 et 3 %, plus préférentiellement entre 1 et 2%.

Selon un mode de réalisation avantageux de l'invention, le procédé comporte, de plus, une étape de :
- dépigmentation des algues avec ou sans traitement par un agent chimique, suite à l'étape de récolte d'une biomasse d'algues et préalablement à la réduction de la quantité intrinsèque de protéines des algues récoltées.

La dépigmentation a pour objectif de blanchir et, ou, de réduire le taux de pigments naturels, par exemple de chlorophylle, de l'algue.

La dépigmentation naturelle des algues peut être obtenue par exemple en répartissant les algues en couches à l'extérieur de tout bâtiment durant la journée et en les lavant ensuite durant la nuit. Ces étapes peuvent être répétées durant approximativement trois jours.

Pour une dépigmentation chimique, on utilisera par exemple un agent de blanchiment chloré.

De manière préférée, la dépigmentation sera réalisée par extraction alcoolique, au moyen d'une ou plusieurs étapes de macérations de la biomasse dans des solutions d'alcool, avantageusement d'éthanol.

Selon un mode de réalisation de l'invention, le procédé comporte, de plus, directement après l'étape de réduction de la quantité de protéines intrinsèque des algues :
- une étape de déstructuration des parois cellulaires des algues, par mélange du résidu d'algues et d'un agent solubilisant des polysaccharides, tel qu'un agent chélatant ou un acide inorganique, préférentiellement choisi parmi l'acide citrique, l'acide citrique monohydraté, l'oxalate de sodium, l'acide chlorhydrique, le carbonate de sodium, plus préférentiellement l'acide citrique, chauffage du mélange à une température comprise entre 80 et 100°, préférentiellement 90°C, pendant 1 à 4h, préférentiellement 2h, puis refroidissement et concentration du résidu d'algues, étant entendu que la dite étape ne comporte aucune élimination ou séparation des composants organiques et inorganiques.

Une telle étape permet d'obtenir un extrait de polysaccharides dits « semi raffinés » ou solubilisés. Au cours de cette étape, les agents solubilisants chélatants, tels que l'acide citrique ou l'acide oxalique, vont capter certains ions présents dans la paroi cellulaire et favoriser ainsi la solubilisation de certains polysaccharides ioniques et la déstructuration des parois cellulaires. De manière alternative, lorsque l'agent solubilisant est un agent inorganique tel que l'acide chlorhydrique, celui-ci va modifier également la solubilité des polysaccharides, voire les hydrolyser partiellement, et modifier les interactions au sein de la paroi cellulaire. Ils produisent ainsi également un effet de déstructuration des parois cellulaires.

Dans le résidu obtenu, les polysaccharides sont solubilisés pour une grande partie, mais ne sont pas séparés des autres constituants non polysaccharidiques de l'algue. Le résidu obtenu à l'issue de cette étape contient en effet tous les composants de l'algue présents avant sa mise en oeuvre, c'est à dire, les polysaccharides semi raffinés, les fibres, le reste de la matière protéique non éliminée à l'étape précédente. Il n'y a pas d'extraction/séparation/purification des polysaccharides comme dans le cas d'un extrait de polysaccharide « raffiné ». Cette étape rend les polysaccharides, notamment pariétaux, accessibles et fonctionnels par comparaison avec les mêmes polysaccharides contenus dans des cellules aux parois structurées. Pour cette raison, on utilisera également dans la présente demande l'expression « algue activée » ou « étape d'activation de l'algue ». Cette étape est avantageuse en termes de coût et de temps, car elle n'intègre pas un procédé d'extraction total des polysaccharides notamment pariétaux, dit procédé raffiné, mais un procédé de semi extraction ou procédé semi raffiné.

De manière avantageuse, l'étape de déstructuration des parois cellulaires des algues comporte les étapes successives de :
- obtention d'un broyat de la biomasse algale,
- mélange dudit broyat et d'eau pour obtenir une pâte,
- ajout d'un agent solubilisant, en particulier l'acide citrique, jusqu'à un pH compris entre 2,5 et 3,5, préférentiellement 3,
- chauffage du mélange à une température comprise entre 80 et 100°, préférentiellement 90°C, pendant 1 à 4h, préférentiellement 2h,
- refroidissement à une température inférieure ou égale à 45°C,
- concentration sous vide à une température comprise entre 45 et 50°C,
- ajustement du pH du résidu obtenu à une valeur comprise entre 7 et 8, préférentiellement 7,7 à l'aide d'une base telle que de l'hydroxyde de sodium.

Le choix de l'agent solubilisant des polysaccharides doit tenir compte de la compatibilité entre cet agent et tout autre composé qui sera ajouté dans la préparation des compositions de bioplastiques, tels que les plastifiants. Les demandeurs ont ainsi mis en évidence que le poids moléculaire de l'agent solubilisant était un élément prépondérant de manière à ce que cet agent puisse être par exemple thermo stabilisant, comme cela sera illustré dans ce qui suit.

Selon un mode de réalisation, le procédé selon l'invention comporte, de plus, après l'étape de réduction de la quantité de protéines intrinsèque des algues et, lorsque le procédé comprend une étape de déstructuration des parois cellulaires des algues, directement après celle-ci, une étape d'obtention d'une pâte comprenant les étapes de :
- précipitation à l'aide d'alcool,
- séparation alcool et précipité,
- séchage du précipité.

Selon un mode de réalisation préféré de l'invention, le procédé comporte, de plus, immédiatement avant l'étape d'obtention d'une pâte, une étape d'ajout d'amidon dans le résidu d'algues comprenant les étapes de :
- mélange d'une dispersion d'amidon et du résidu d'algues préalablement broyé,
- chauffage du mélange à une température comprise entre 80 et 100°C durant 1 à 3 h,
- refroidissement jusqu'à une température comprise entre 45 et 50°C,

L'amidon est un composé d'origine naturelle et biodégradable qui est aujourd'hui considéré comme une matière première intéressante pour la production de matières plastiques biobasées. Il peut être plastifié facilement à l'aide d'un plastifiant, tel de l'eau ou du glycérol, et convient particulièrement à la fabrication de films.

L'amidon peut être choisi parmi les amidons natifs tels que l'amidon de maïs, l'amidon de blé, l'amidon de pomme de terre, l'amidon de tapioca, l'amidon de pois, l'amidon de riz ou leurs mélanges, ou encore d'un polymère dérivé de l'amidon.

De manière avantageuse, le procédé comporte une étape de culture des algues en conditions favorisant la biosynthèse d'amidon, ladite culture comprenant l'installation en bassin d'une biomasse d'algues et la culture durant quatre à six semaines, préférentiellement quatre semaines, dans un milieu de culture d'eau de mer sans apports en fertilisants, notamment sans apports en azote.

Une biomasse d'algues contenant jusqu'à 30 %, voire jusqu'à 40 % d'amidon, en poids par rapport au poids de la biomasse, peut être obtenue à partir de conditions de culture favorisant la synthèse d'amidon par l'algue. De telles conditions d'aquaculture sont définies dans la publication scientifique intitulée « Tuning the polysaccharide profile in Ulvacea through controlled tank aquaculture conditions », CEVA, Alg'n'Chem , Montpellier, November 2011, ainsi que la thèse de doctorat publiée et intitulée « Influence des conditions de culture d'algues marines de l'ordre des Ulvales sur leur croissance et leur composition », 2009. Dans cette thèse, il est établi que lorsque les algues des espèces Ulvaria obscura et Ulva armoricana sont cultivées en conditions de milieu appauvri, leur teneur en sucres totaux augmente considérablement. Cela est dû à l'augmentation de la teneur en glucose dans les tissus, au détriment des autres sucres constitutifs. Les algues soumises à une limitation en azote se mettent à fabriquer des sucres, et notamment des sucres de réserve tels que l'amidon (Gómez Pinchetti et al., 1998, Andersen, 2005). De même on sait que la dégradation de l'amidon est accélérée à l'obscurité, notamment quand les milieux de culture sont enrichis en azote (Rosenberg et al., 1982 ; Williams et al., 1985 ; Ekman et al., 1991; Rincones et al., 1993; Wiencke et al., 2007). Ainsi, en condition de cultures contrôlées, c'est à dire de contrôle de la durée de culture, de la luminosité et de l'enrichissement ou non du milieu de culture à l'aide d'une composition fertilisante, notamment supplémentant le milieu en azote, il est possible d'influencer le métabolisme des algues de manière à aboutir à la synthèse de certains composés. En ce qui concerne l'augmentation de la synthèse d'amidon par l'algue, il a donc été constaté qu'il était utile de ne pas supplémenter le milieu de culture en fertilisants, notamment en azote. De plus, l'absence d'obscurité lors de la culture est à favoriser. Néanmoins afin de permettre d'obtenir une forte biomasse présentant une teneur en amidon jusqu'à 40% de la matière sèche, les conditions de cultures peuvent être définies comme ceci : une première phase de culture en présence d'eau de mer supplémentée en matières fertilisantes (composition de Conway ou Walne), de manière à favoriser la croissance de la biomasse, suivie d'une phase de culture en présence d'eau de mer non supplémentée en matières fertilisantes, notamment en azote, de manière à favoriser la production d'amidon par l'algue. De manière pratique, les algues sont récoltées dans un milieu marin riche en nutriments. Il peut s'agir, par exemple, d'une zone connue de prolifération des algues. Les algues sont ensuite transférées dans des bassins dépourvus de nutriments dans lesquels elles vont alors transformer les protéines en amidon ce qui correspond à un enrichissement de l'amidon pour une perte en taux de protéines. On obtient par exemple plus de 20% (en poids) de glucose au bout de quatre jours de maturation et près de 40 % au bout de neuf jours.

Selon un mode de réalisation de l'invention, les algues sont des microalgues ou des macroalgues, préférentiellement des algues vertes (chlorophyceae), brunes (Phaeophyceae) ou rouges (Rhodophyceae), plus préférentiellement des algues du genre Ulva ou de la famille des Sargassaceae.

A titre d'exemple d'algues vertes du genre Ulva, on citera Ulva armoricana ou ulva lactuca.

La distinction entre microalgue et macroalgue est imprécise. Les algues classées parmi les macroalgues sont les algues dont l'appareil végétatif est visible à l'œil nu, celui des microalgues étant visible au moyen d'un microscope.

Les microalgues pourront être par exemple choisies parmi les chlorophycées ou les diatomées.

L'invention concerne encore une poudre d'algues obtenue à l'aide d'un procédé tel que décrit précédemment, ainsi qu'une composition bioplastique, notamment destinée à la fabrication d'un produit plastique, comprenant une poudre d'algues obtenue à l'aide d'un procédé tel que décrit précédemment.

Avantageusement, la poudre d'algues selon l'invention contient moins de 5% de protéines, en masse de protéines par rapport à la masse totale de la poudre. Généralement il permet d'obtenir une poudre contenant entre 1 et 4%, préférentiellement entre 1 et 2% de protéines par rapport à la quantité totale de poudre.

Le procédé selon l'invention permet ainsi l'obtention d'une poudre d'algues à forte valeur ajoutée étant donné la faible quantité de protéines dans la poudre obtenue.

Avantageusement, la composition bioplastique comprend au moins un autre composant tel qu'un composé plastifiant, un polymère naturel, un stabilisant, un antioxydant, un anti ultra-violet, un colorant, une charge, un agent favorisant la compatibilité entre composés, de l'eau ou un conservateur.

Les plastifiants, apportant davantage de flexibilité et favorisant la plastification de la poudre d'algues, ont été sélectionnés parmi les polyols à partir de triglycérides végétaux comme par exemple les triols : glycérine (monoglycérol), diglycérines ( diglycérol >90%), les polyglycérines 3 (triglycérol >35%), les polyglycérine 4 ( tri et tétraglycérol >65% ); les diols : éthylene glycol, diéthylène glycol, triéthylène glycol , propylène glycol (propane -1,2 diol) , triméthylène glycol (propane 1,3 diol) , butylène glycol (butane -1,3 diol ), n-butylène glycol (butane -1,4 diol ), 2,3-butylène glycol ou secbutylène glycol (butane-2,3-diol ) ; les tertaols :erythritol ; les pentols : Xylitol , Arabitol , Ribitol ; les hexols : sorbitol , galactitol , mannitol ; les heptols : Volemitol ; Les Disaccharides polyols à neuf fonctions OH : Maltitol, isomaltitol , lactitol et toute combinaison de ces composés.

Ont été utilisés préférentiellement, les diglycérines végétales sans OGM, telles que le produit SP-PG3 de SPIGA. Ils seront d'ailleurs préférés à la glycérine car moins migrants. En effet, la glycérine a tendance à migrer en surface de la pièce plastique réalisée et tend à procurer un aspect gras au produit, avec une perte des propriétés mécaniques. La molécule de diglycérol est plus encombrante que celle de la glycérine et migre donc beaucoup moins dans la matrice polymérique. En outre, les diglycérines assurent une meilleure plastification des bioplastiques. Ces plastifiants sont utilisés à des taux de 10 %, en poids, de la poudre d'algues et jusqu'à 10% du bioplastique.

Des polymères autres que l'amidon peuvent être ajoutés et sont choisis en fonction des caractéristiques qu'ils peuvent apporter au produit plastique recherché. Il peut s'agir de caractéristiques d'hydrophobicité, de transparence, de résistance à la traction, la dureté, la capacité à permettre l'impression, etc...

Des polymères thermoplastiques biodégradables sont choisis parmi l'acide polylactique (PLA) (produit par la société Natureworks), le polybutylene succinate (PBS), le polybutylene succinate adipate ( PBSA) (produits par la société Showa Denko sous les references BIONOLLE serie 1000 ( PBS ) et 3000 (PBSA) ), le polybutylene adipate téréphtalate (PBAT copolyester) (produit par la société BASF sous les références ECOFLEX ou la société DuPont sous la référence BIOMAX), les polyhydroxyalcanoates (PHA) (produits par la société METABOLIX), les polyhydroxybutyrates (PHB), les polyhydroxyvalérates (PHV), les poly hydroxybutyratehydroxyvalérates copoplymères (PHBV), les polycaprolacones ( PCL) (société CAPA, série 6500 and 6800).

Des alcools polyvinyliques, ou PVA, peuvent être mélangés avec la poudre d'algues dans les compositions de la présente invention et renforcent la biodégradabilité, les propriétés de transparence et de résistance mécanique du matériau fini, la flexibilité et permettent une bonne dispersion des amidons. Les PVA seront choisis avec des poids moléculaires entre 20 000 à 80 000 Da. Parmi les PVA disponibles sur le marché, nous pouvons citer le produit commercialisé sous la marque CELVOL E 205 de la société Sekishui; 87-90% hydrolysé, présentant un poids moléculaire entre 30 000 - 70 000 Da et une viscosité comprise entre 4-6 cps à 4%, ou le produit commercialisé sous la marque MOWIOL 5-88 de la société Kuraray ; 87% hydrolysé, avec un poids moléculaire d'environ 31 000 Da.

Des compositions bioplastiques issues de matières biosourcées peuvent également être produites en mélangeant la poudre d'algues avec des polyoléfines produits à partir de ressources végétales comme les polyéthylènes issus de canne à sucre, tels que ceux de la société BRASKEM.

Des compositions bioplastiques hybrides peuvent inclure des poudres d'algues et des polymères non-biodégradables et non biosourcés tels que les polyoléfines (polypropylène homopolypropylène (Homo PP) et copolymère de polypropylène(CoPP), les polyéthylènes), les polystyrènes, les polyesters, le Polychlorure de Vinyl (PVC), poly (acrylonitrile-co-butadiene-co -styrène (ABS), les élastomères thermoplastiques comme les Polyurethanes , les copolymères block styrènique ( SAS, SBS ) tels que ceux produits par KRATON polymers LLC , houston , Texas, l'éthylène vinyle acétate (EVA), ou leur mélanges.

Les compositions bioplastiques peuvent également inclure des additifs tels que des anti ultraviolets et antioxydants. Parmi ceux-ci on citera les produits commercialisés sous la marque Irganox 1010, Irganox B-225, Irganox B-900, irgastab de la société Ciba specialty chemicals, Cyanox LTDP de la société Cytec. Ces additifs sont ajoutés dans des quantités pouvant aller jusqu'à 0,5%, en poids du poids total de la composition.

A titre de charges minérales, on citera le talc, le carbonate de calcium, le dioxyde de titane (rutile ou anatase). Ces charges sont utilisées pour apporter de la blancheur et ou de l'opacité au produit plastique final. On peut également utiliser le noir de carbone qui donnera de l'opacité et la couleur noire au produit plastique final.

Les compositions bioplastiques peuvent également inclure des additifs oxobiodégradables thermo réactifs et/ou photo réactifs qui, sous l'effet de la lumière, de la température ou de la combinaison des deux, vont oxyder les polyoléfines tels que polyéthylènes et polypropylènes pour les rendre biodégradables en présence de microorganismes. A titre d'exemple, de tels additifs sont produits par la société Symphony dans la série D2W - DG13-15.

Des absorbeurs d'odeurs pourront être ajoutés également, pour une action supplémentaire sur les problèmes d'odeur dans la formation des bioplastiques, lorsque des additifs particulièrement odorants seront ajoutés tels que la vanilline (masqueur d'odeur) ou des additifs antimicrobiens inorganiques dans des matrices PE tels les masterbatch 9655 de la société Symphony.

De manière préférée, la composition comprend entre 10 et 60%, en poids, préférentiellement entre 15 et 55 %, en poids, d'une poudre d'algues obtenue à l'aide d'un procédé tel que défini précédemment et entre 90 et 40 % d'au moins un autre composant.

La présente invention concerne encore un procédé de fabrication d'un produit plastique qui inclut un procédé de préparation d'une poudre d'algues tel que défini précédemment.

Avantageusement, le procédé de fabrication dudit produit plastique comprend, de plus, une étape de préparation d'une composition bioplastique à partir de ladite poudre d'algues.

Avantageusement encore, le procédé de fabrication dudit produit plastique comprend une étape d'extrusion de ladite composition bioplastique en granulés.

Avantageusement encore, le procédé de fabrication dudit produit plastique comprend une étape de mise en forme d'un produit plastique par moulage par injection, extrusion soufflage ou extrusion en filière plate de ladite composition bioplastique.

L'invention concerne enfin un produit plastique obtenu à partir d'un tel procédé de fabrication.

Selon un mode de réalisation préféré de l'invention, le produit est un film alimentaire ou non alimentaire, un objet moulé ou thermoformé, tel qu'un emballage par exemple multi-couches ou en feuilles.

Les caractéristiques de l'invention, ainsi que d'autres, apparaîtront plus clairement à la lecture des modes de réalisations suivants qui se veulent illustratifs et non restrictifs.

### Exemple 1 : Obtention d'une poudre d'algues témoin :

Des algues vertes de l'espèce Ulva Armoricana ont été récoltées, séchées en flocons à 50°C, puis réduites en particules de taille moyenne de 100 µm de diamètre. Une poudre de 2 Kg a ainsi été obtenue. La composition de la poudre d'algues est donnée dans le Tableau 1.

**Tableau 1**

| | |
|---|---|
| Matière sèche (MS) | 88,9 % sec |
| Teneur en amidon | 4,1 % sec/brut^{∗} |
| Teneur en protéines | 14,5% sec/brut |

| | |
|---|---|
| ^{∗}% sec/brut : masse de matière sèche du composé par rapport à la masse des algues séchées « brutes », c'est-à-dire sans correction du pourcentage d'humidité résiduelle encore présent dans l'algue séchée. | |

On note que le taux d'amidon est de 4,1% sec/brut dans cette poudre.

### Exemple 2 : Obtention d'une poudre d'algues témoin, dite activée et enrichie en amidon :

Le procédé utilisé met en œuvre, dans l'ordre, les étapes suivantes, détaillées dans l'exemple 3 à suivre:
1- récolte d'une biomasse d'algues vertes Ulva Armoricana et enrichissement en conditions favorisant la synthèse d'amidon,
2- dépigmentation,
3- **absente** (étape de réduction du taux de protéines intrinsèque non réalisée),
4- déstructuration des parois cellulaires par traitement à l'acide citrique (étape d'activation des algues),
5- ajout d'amidon (mélange final de 60% massique sec d'algues activées et 40 % massique sec d'amidon),
6- précipitation/filtration,
7- séchage/ réduction en poudre de taille moyenne de 100 µm de diamètre.

Les étapes 1, 2, 4, 5 et 6 se déroulent donc comme celles de l'exemple 3 à suivre.

### Exemple 3 : Obtention d'une poudre d'algues selon l'invention, dite activée, déprotéinée et enrichie en amidon :

Le procédé utilisé met en oeuvre, dans l'ordre, les étapes suivantes, développées dans ce qui suit :
1 - récolte d'une biomasse d'algues vertes Ulva armoricana et culture en conditions favorisant la synthèse d'amidon,
2 - dépigmentation,
3 - réduction du taux de protéines intrinsèque,
4 - déstructuration des parois cellulaires par traitement à l'acide citrique (étape d'activation des algues),
5 - ajout d'amidon,
6 - précipitation/séparation,
7 - séchage et réduction en poudre de taille moyenne de 100 µm de diamètre.

### 1. Récolte et culture de biomasse en condition d'enrichissement en amidon :

Les ulves, de l'espèce Ulva armoricana, ont été récoltées en France sur une période s'étalant entre septembre et octobre. A une telle période, elles possèdent des teneurs initiales élevées en glucose favorables à un enrichissement endogène en amidon. Les algues ont été cultivées durant 1 mois et 4 jours en bassin et en conditions de privation d'azote. Les conditions de luminosité découlent de l'éclairage naturel des mois d'octobre et novembre en Bretagne (France). Une quantité de 18 kg de matière fraiche a été obtenue. Les algues récoltées ont été congelées sous vide, sans rinçage à l'eau douce.

15 kg d'algues fraiches ont été par la suite décongelées et broyées par broyeur de type URSCHEL sur grille 66896. Après décongélation et broyage, 14,65 kg d'algues broyées ont été obtenues. Les données suivantes ont été mesurées :
Taux de matière sèche : 21,38 %
Masse d'algues (MS: matière sèche) : 3,13 kg
Teneur en protéines : 9,29 % sec/sec, soit 0,29 kg de protéines
Teneur en glucose (amidon) : 32,80 % sec/sec

La teneur en glucose est représentative de la teneur en amidon, l'amidon étant transformé en glucose lors des analyses quantitatives et qualitatives. On note que le taux de glucose est de 32,80 %sec/sec, par rapport au taux de glucose de l'exemple 1 de 4,1 %sec/brut. Il y a donc bien eu stimulation et augmentation de la synthèse d'amidon par l'algue lors de sa culture dans des conditions favorables à la synthèse de l'amidon par l'algue.

### 2. Dépigmentation à l'éthanol :

Après broyage, réalisation de quatre macérations successives, hydroéthanoliques, dans le but d'enlever un maximum de chlorophylle et de blanchir l'algue. Chaque macération est réalisée par trempage des algues broyées durant 1 à 3 jours en présence de 25 L d'éthanol. A l'issue de ces étapes de macération, le mélange est filtré sur une toile présentant des pores de 100 microns de diamètre et les algues broyées et dépigmentées sont récupérées. La quantité d'ulves décolorées obtenue est de 14,90 kg ayant une MS de 16,47 %, soit 2,45 kg sec. Le rendement massique de cette étape est de 78 %.

La teneur en protéines totales est déterminée par la méthode Kjeldhal (Nx6,25). Le principe de cette méthode est une multiplication de la teneur en azote minéral par un coefficient moyen qui représente la richesse en azote des protéines animales ou végétales. La teneur en protéines totale est exprimée en pourcentage de protéines (masse de matière sèche) par rapport à la masse sèche des ulves dépigmentées séchées « brutes », c'est-à-dire sans correction des quelques pourcentages d'humidité présents dans l'algue séchée.

### Bilan :

Quantité de matière sèche des algues au début de l'étape : 3,13 kg
Quantité de matière sèche des algues à l'issue de l'étape : 2,45 kg
Teneur en protéines : 10,4 % sec/brut, soit 0,25 kg de protéines
Teneur en amidon : 32,1 %sec/brut

### 3. Réduction du taux de protéines intrinsèque :

Le principe de cette étape est d'hydrolyser les protéines par voie enzymatique et d'extraire les hydrolysats en milieu basique. 13,65 kg d'ulves dépigmentées ont été utilisées (taux de MS 16,47 %), soit 2,24 kg sec.

### 3.1 Macération aqueuse

Mise en suspension de 13,65 kg d'ulves dépigmentées issues de l'étape précédente, soit 2,24 kg sec, dans 47,8 kg d'eau déminéralisée et agitation à la turbine Rayneri pendant 30 minutes (MS 3,57 %). Puis ajout de 11,68 kg d'eau déminéralisée pour obtenir 3,02 % MS.

Macération une nuit à une température de 7°C, puis séparation sur tamis 100 µm en statique. Dans un second temps, pressage manuel du résidu d'algue restant sur le tamis 100 µm avec une toile 30 µm.

Bilan de cette sous-étape : quantité : 57 kg (MS : 0,47%) de filtrat et 14,8 kg (MS : 13,04%) soit 1,92 kg sec de résidu d'algues. Le rendement massique intermédiaire de cette étape est de 85 %.

### 3.2. Première extraction à la soude :

Cette étape favorise l'extraction et l'accessibilité à l'enzyme. Elle permet l'extraction des protéines solubles pour favoriser l'action de l'enzyme spécifiquement sur les protéines insolubles.

Le résidu issu de la sous-étape 3.1 est repris (1,92 kg sec) dans de l'eau déminéralisée et mis en suspension à 3,5 % MS. Ensuite, ajout de soude à 30 %, en quantité suffisante pour être en solution finale à 0,12 M en concentration.

Agitation à la turbine Rayneri pendant 1h30.

Séparation sur tamis à 100 µmen statique (égouttage une nuit à température ambiante), puis pressage manuel du résidu d'algues restant sur le tamis 100 µmavec une toile 30 µm.

Bilan de cette sous-étape : quantité : 40,2 kg (MS : 1,04%) de filtrat et 14,7 kg (MS : 12,68%) soit 1,86 kg sec de résidu d'algues. Le rendement massique intermédiaire de cette étape est de 96 %.

### 3.3. Hydrolyse enzymatique avec une protéase Alcalase^{®} :

Reprise du résidu d'algues de la sous-étape 3.2 (1,86 kg sec) dans de l'eau déminéralisée pour une mise en suspension à 3,5 % MS soit 47,43 kg d'eau déminéralisée. Transfert du milieu dans une boule de concentration puis ajustement du pH à 8,0 avec 140 g d'acide sulfurique à 96% (pH départ 12,3). Agitation sur agitateur à vitesse maximale et chauffage à 55° C. Une fois à température, ajout de 3,28 g d'enzyme Alcalase^{®} de la société Novozymes, une protéase issue de *Bacillus lichenformis* (Ref Sigma : P4860, ≥2.4 U/g). Généralement, l'enzyme est ajoutée à une concentration comprise entre 0,1 et 10 % par rapport à la masse sèche de protéines, préférentiellement entre 0,8 et 3 %, plus préférentiellement entre 1 et 2%. Agitation une nuit (12 h) à 55 °C.

### 3.4. Seconde extraction à la soude :

Après refroidissement à 30°C, ajustement du pH à 12,0 avec 498 g de NaOH à 30% (pH initial : 5,23). Séparation sur tamis à 100 µmen statique, puis dans un second temps, pressage manuel des résidus d'algues restant sur le tamis 100 µmavec une toile 30 µm.

Bilan de cette étape : quantité : 47 kg (MS : 1,97%) de filtrat et 14 kg (MS : 8,90%) soit 1,24 kg sec de résidu d'algues. Le rendement massique intermédiaire à cette étape est de 66 %.

Le filtrat, riche en matière protéique (peptides et acides aminés) est récolté dans un but de valorisation ultérieure.

### 3.5. Rinçage à l'eau du résidu d'algues

Reprise du résidu obtenu de la sous-étape 3.4 (1,24 kg sec) et rinçage à l'eau déminéralisée pour une mise en suspension à 3 % MS, soit 27,53 kg d'eau déminéralisée rajoutée.

Agitation une heure et séparation sur tamis 100µm pendant une nuit (aucun pressage sur toile 30µm nécessaire).

### Bilan :

Quantité de matière sèche des algues au début de l'étape : 2,24 kg
Quantité de matière sèche des algues à l'issue de l'étape : 1,11 kg
Rendement massique : 49 %.
Teneur en protéines : 5,6 % sec/brut, soit 0,06 kg de protéines
Teneur en amidon : 49,4 %sec/brut

La teneur en protéines du résidu d'algues déterminée par la méthode Kjeldhal (Nx6,25) est de 5,6% sec/brut. La teneur en protéines dans le résidu (5,6% sec/brut) a été réduite de 46% par rapport à la teneur en protéine dans le résidu de l'étape précédente (10,4% sec/brut).

Par rapport à la quantité de protéines totales de départ (0.29 kg), la quantité de protéines finale (0.06 kg) a été réduite de 79% en masse.

### Hydrolyse enzymatique à partir d'une autre enzyme :

Une hydrolyse enzymatique des protéines a été réalisée, à titre comparatif, sur une biomasse de 2 kg afin de vérifier la faisabilité du procédé à l'aide d'autres protéases, notamment à partir d'une protéase dérivée *d'Aspergillus oryzae* et commercialisée sous le nom Flavourzyme par la société Sigma. La biomasse algale utilisée a subi dans ce test les mêmes étapes que les étapes 1 à 3 décrites précédemment. La teneur en protéines a été réduite de 12 % (quantité de protéines résiduelles (en masse), par rapport à la quantité de protéines présentes initialement.

Les caractéristiques de couleur sont l'obtention d'un résidu jaune-vert comme dans le cas de l'utilisation d'Alcalase. Cependant une odeur peu satisfaisante est relevée. L'hydrolyse à l'aide de l'Alcalase est préférée.

### 4. Activation des algues : déstructuration des parois cellulaires :

Pour rappel, le principe est de déstructurer les parois cellulaires à l'aide d'un agent solubilisant des polysaccharides, dans le cas présent à l'aide d'un agent chélatant, et plus précisément de casser les liaisons notamment ioniques impliquant les polysaccharides pariétaux de manière à rendre les polysaccharides pariétaux accessibles, libres et fonctionnels, donc actifs, sans être obligés de les extraire au moyen de procédés d'extraction raffinés et fins. A l'issue de cette étape, le résidu d'algues récupéré contient les polysaccharides ainsi que tous les composants non polysaccharidiques présents avant la mise en oeuvre de cette étape.

Le résidu d'algues issu de l'étape 3 est repris pour être broyé au broyeur colloïdal serré au maximum pour avoir une pâte lisse, avec un ajout de 12 kg d'eau afin de pousser les résidus et rincer le broyeur. Après broyage, obtention de 26,6 kg (MS : 4,07%), d'un résidu d'algues sous forme d'une purée épaisse, soit 1,08 kg sec.

Transfert du résidu d'algues broyé, soit 26,6 kg, dans un réacteur émaillé de contenance de 100 L et ajout de 11,4 kg d'eau pour rinçage du matériel. Au total 38 kg de broyat (MS finale calculée : 2,85%), soit 1,08 kg sec, engagés pour l'activation à l'aide d'un agent chélatant : l'acide citrique monohydraté.

Le pH est ajusté à 3 avec 436,5 g d'acide citrique monohydraté.

Le milieu réactionnel est chauffé et maintenu pendant 2 h à 90°C sous agitation, puis refroidi à température ambiante (mesure du pH après refroidissement : 3,06).

Ensuite, la totalité du milieu (résidu d'algues et liquide) est transférée directement dans une boule de concentration et concentrée sous vide à 45 à 50°C. Environ 15 litres d'eau sont alors éliminés. On note à ce stade l'absence d'étape de séparation ou filtration aboutissant à l'élimination des composés non polysaccharidiques.

22,2 kg, à 6,31% MS, soit 1,4 kg sec de concentré sont récupérés.

Neutralisation du concentré à pH 7,7 avec 510 mL de NaOH 30% (MS après neutralisation : 6,78 %), soit 1,5 kg sec.

Le rendement intermédiaire concernant cette étape globale d'activation est considéré comme 100 % car aucune source de perte n'est identifiée (1,08 kg + 0, 487 kg de citrate de sodium).

### Bilan :

Quantité de matière sèche des algues au début de l'étape : 1,11 kg
Quantité de matière sèche des algues à l'issue de l'étape : 1,54 kg
Teneur en protéines : 4 % sec/brut
Teneur en amidon : 34,4 %sec/brut

### 5- Ajout d'une dispersion d'amidon

Le concentré d'algues activées issu de l'étape précédente est utilisé à raison de 22,2 kg (6,78 %) soit 1,5 kg sec.

Une dispersion d'amidon a été préparée de façon à obtenir un mélange final de 60% massique sec d'algues activées et 40 % massique sec d'amidon. Pour éviter d'ajouter trop d'eau, préparation d'une dispersion à environ 10% d'amidon soit 8,29 kg d'eau et 1,01 kg d'amidon. La dispersion d'amidon est maintenue à 90°C pendant 30 minutes (cuisson). La dispersion est très visqueuse, 10% est une limite qu'il est préférable de ne pas dépasser.

Transfert du concentré d'algues activées et de la dispersion d'amidon à 90 °C dans un contenant permettant une agitation au Rayneri. Agitation pendant trois heures pour bien mélanger puis transfert des 34 kg de dispersion dans 102 L d'alcool (80 L d'alcool neuf et 22 L d'alcool recyclé à 90°).

Mélange puis repos de la solution une nuit pour décantation. Séparation du précipité sur une toile de 30 µm puis pressage manuel sur toile de 20µm. La quantité de mélange pressé récupéré est de 13,1 kg.

Mise en séchage dans l'étuve, chauffage 5h à 45 °C. Le rendement de cette étape est de 152 %.

### Bilan :

Quantité de matière sèche des algues au début de l'étape : 1,5 kg
Quantité de matière sèche des algues à l'issue de l'étape : 2,3 kg
Teneur en protéines : 2,4 % sec/brut, soit 0,05 kg de protéines
Teneur en amidon : 53,4 %sec/brut

### 6. Broyage :

Un premier broyage sur broches Forplex a lieu, suivi d'un deuxième broyage sur grille 100 µmForplex.

Quantité après broyage : 1,91 kg.

Rendement broyage : 83 % massique, liés aux volumes morts des broyeurs.

La répartition granulométrique de ces algues broyées est la suivante :

| | |
|---|---|
| > 250 µm | 0% |
| 250 µm - 160 µm | 0,48 % |
| 160 µm - 80 µm | 20% |
| 80 µm - 40 µm | 75,23 % |
| < 40 µm | 4,29 % |

On note qu'à l'issue du procédé, on obtient une poudre d'algues contenant 2,4 % de protéines et 53 % d'amidon, en pourcentage de masse sèche par rapport à la masse sèche totale de la poudre.

### Exemple 4: compositions bioplastiques :

Des compositions de bioplastiques ont été formulées par mélange des poudres d'algues des exemples 1, 2 et 3 avec d'autres composés. A titre de témoin, de l'amidon de maïs natif est utilisé. Les compositions formulées sont extrudées sous forme de granulés. Au total 49 compositions ont été formulées. Le Tableau 2 ci-dessous indique les compositions des mélanges réalisés :

**Tableau 2 :**

| **Formule** | Type de poudre | | | | Plastifiant | Polymère |
|---|---|---|---|---|---|---|
| N° | Amidon (témoin) | (exemple 1) | (exemple 2) | (exemple 3) | Diglycérine | PBAT |
| 1 | 35% | | | | 5% | 60% |
| 2 | 45% | | | | 7% | 48% |
| 3 | 55% | | | | 8% | 37% |
| 4 | | 25% | | | 4% | 71% |
| 5 | | 35% | | | 5% | 60% |
| 6 | | 45% | | | 7% | 48% |
| 7 | | 55% | | | 8% | 37% |
| 16 | | | 25% | | 4% | 71% |
| 17 | | | 35% | | 5% | 60% |
| 18 | | | 45% | | 7% | 48% |
| 19 | | | 55% | | 8% | 37% |
| **34** | | | | 25% | 4% | 71% |
| **35** | | | | 35% | 5% | 60% |
| **36** | | | | 45% | 7% | 48% |
| **37** | | | | 55% | 8% | 37% |

| | | | | | | |
|---|---|---|---|---|---|---|
| PBAT : (polybutylene adipate terephtalate) | | | | | | |

Suite :

| **Formule** | Type de poudre | | | | Plastifiant | |
|---|---|---|---|---|---|---|
| | Amidon | Exemple 1 | Exemple 2 | Exemple 3 | Diglycérine | Polymère CoPP |
| 8 | | 25% | | | 4% | 71% |
| 9 | | 35% | | | 5% | 60% |
| 10 | | 45% | | | 7% | 48% |
| 11 | | 55% | | | 8% | 37% |
| 22 | | | 25% | | 4% | 71% |
| 23 | | | 35% | | 5% | 60% |
| 24 | | | 45% | | 7% | 48% |
| 25 | | | 55% | | 8% | 37% |
| **40** | | | | 25% | 4% | 71% |
| **41** | | | | 35% | 5% | 60% |
| **42** | | | | 45% | 7% | 48% |
| **43** | | | | 55% | 8% | 37% |

| | | | | | | |
|---|---|---|---|---|---|---|
| CoPP : copolymère de polypropylène | | | | | | |

Suite

| **Formule** | Type de poudre | | | | Plastifiant | |
|---|---|---|---|---|---|---|
| | Amidon | Exemple 1 | Exemple 2 | Exemple 3 | Diglycérine | Polymère PBS |
| 12 | | 25% | | | 4% | 71% |
| 13 | | 35% | | | 5% | 60% |
| 14 | | 45% | | | 7% | 48% |
| 15 | | 55% | | | 8% | 37% |
| 28 | | | 25% | | 4% | 71% |
| 29 | | | 35% | | 5% | 60% |
| 30 | | | 45% | | 7% | 48% |
| 31 | | | 55% | | 8% | 37% |
| **46** | | | | 25% | 4% | 71% |
| **47** | | | | 35% | 5% | 60% |
| **48** | | | | 45% | 7% | 48% |
| **49** | | | | 55% | 8% | 37% |

| | | | | | | |
|---|---|---|---|---|---|---|
| PBS : Polybutylène succinate | | | | | | |

Les formules 34 à 37, 40 à 43 et 46 à 49 comprennent entre 25 et 55% en poids, par rapport au poids total des compositions, d'une poudre d'algue obtenue selon l'exemple 3. Il s'agit d'algues dont le taux de protéines a été réduit, le taux d'amidon intrinsèque a été augmenté, un apport d'amidon a été ajouté et les polysaccharides pariétaux ont été rendus fonctionnels.

### Exemple 5 : Extrusion sous forme de granulés des compositions de l'exemple 4

Les conditions d'extrusions sont indiquées dans le Tableau 3

**Tableau 3 :**

| **Formule** | | Conditions d'extrusion | | Injection |
|---|---|---|---|---|
| 1-3 | | 160, 170, 180°C - 100 rpm | | 170°C - 8 bars |
| 4-7 | | 140, 170, 180°C - 100 rpm | | 170°C - 8 bars |
| 8-10 | | 140, 170, 180°C - 50 rpm | | 170°C - 8 bars |
| 12-15 | 120,140, | 160 °c - 75 rpm | | 160°c - 8 bars |
| 11 | 140 , 190, 200°C - 50 rpm: extrusion difficile | | | 180°C - 8 bars |
| **16** | | 120, 140, 160°C - 50 rpm | | 160°C - 8 bars |
| 17 | | 120, 140, 170°C - 50 rpm | | 170°C - 8 bars |
| 18 | | 120, 160, 180°C - 50 rpm | | 180°C - 8 bars |
| 19 | 130, 160, 180°C - 50 rpm: extrusion difficile | | | 180°C - 8 bars |
| 22-25 | | 140, 170, 180°C - 50 rpm | | 170°C - 8 bars |
| 34-37 | | 120, 140, 170°C - 50 rpm | | 170°C - 8 bars |
| 40-42 | | 140, 170, 190°C - 50 rpm | | 180°C - 8 bars |
| 43 | | Extrusion impossible | | |

Les caractéristiques d'odeur, de couleur et les caractéristiques mécaniques des compositions extrudées sont présentées dans le Tableau 4 :

**Tableau 4 :**

| **Formule** | Odeur | Couleur | Nombre éprouvettes | Propriétés mécaniques | | | | |
|---|---|---|---|---|---|---|---|---|
| | | | | Module Mpa | Contrainte seuil | Déformation au seuil | Contrainte rupture MPA | Déformation rupture % |
| 1 | pain | beige clair | 5 | 99 (3) | 10.4 (0.1) | 31.9 (1.5) | 15.6 (1.2) | 387 (225) |
| 2 | pain | beige | 3 | 143 (2) | 9.9 (0.7) | 18 (2) | 12.1 (1.4) | 269 (67) |
| 3 | pain | beige | 5 | 167 (9) | 10.5 (0.1) | 14.3 (0.5) | 11.7 (0.5) | 186 (35) |
| | | | | | | | | |
| 4 | algues | brun sombre | 3 | 83 (3) | 11.8 (0.2) | 46.4 (2) | 21 (0.3) | 477 (10) |
| 5 | algues | brun sombre | 4 | 100 (2) | 11.7(0.1) | 41.4(1) | 18.4 (0.5) | 458 (27) |
| 6 | algues | brun sombre | 5 | 124 (13) | 11 (0.2) | 36.5 (4) | 13.2 (0.5) | 266 (23) |
| 7 | algues | brun sombre | 4 | 164 (30) | 11.8 (0.7) | 24.5 (5) | 12 (1.1) | 93 (53) |
| 8 | algues | brun sombre | 5 | 600 (41) | 35.5 (0.5) | 25 (2) | 27.2 (0.4) | 86 (19) |
| 9 | algues | brun sombre | 4 | 453 (67) | 33 (2) | 22 (2.3) | 26.5 (1.4) | 55 (19) |
| 10 | algues | brun sombre | 3 | 435 (77) | 33 (5) | 23 (3) | 30.3 (4.8) | 35 (6) |
| 11 | algues | brun sombre | 1 | 567 | 26 | 18 | 23,9 | 42 |
| 12 | algues | Brun sombre | 6 | 566(34) | 32.3(1.4) | 17.8(1.5) | 29.9(1.6) | 29(5) |
| 13 | algues | Brun sombre | 4 | 600(29) | 33.7(1.8) | 17.7(1.3) | 32.3(2) | 28(1) |
| 14 | algues | Brun sombre | 4 | 727(42) | 32.2(1) | 12.7(0.7) | 31.5(1) | 17(1) |
| 15 | algues | Brun sombre | 3 | 826(87) | 26.2(0.6) | 7.6(0.6) | 25.6(0.7) | 10 (0.4) |
| 16 | faible | vert clair | 5 | 92 (13) | 11.3 (0.6) | 34.2 (4.4) | 15.5 (0.5) | 171 (32) |
| 17 | faible | vert clair | 5 | 139 (10) | 10.4 (0.5) | 23.8 (3) | 12.3 (0.5) | 110 (0.5) |
| 18 | faible | vert | 5 | 198 (23) | 9.8 (0.4) | 14.5 (2.1) | 9.7 (0.4) | 56 (13) |
| 19 | faible | vert foncé | 3 | 254 (63) | 9.8 (1.7) | 10.3 (7) | 10.6 (0.3) | 30 (26) |
| 22 | faible | semi transparente | 3 | 418 (16) | 38.1 (1.3) | 25.3 (1.7) | 28.8 (1.4) | 124 (45) |
| 23 | faible | beige clair | 3 | 640 (15) | 32.1 (1.7) | 25 (0.1) | 25.1 (0.9) | 75 (1) |
| 24 | faible | beige | 4 | 713 (74) | 26.6 (1.8) | 23 (2) | 24.3 (2.2) | 67 (28) |
| 25 | faible | beige foncé | 3 | 941 (26) | 21.4 (1) | 17 (0.1) | 21.5 (1) | 24 (0.8) |
| 28 | faible | vert clair | 3 | 402(4) | 30(1.5) | 32(2) | 31.5(1.4) | 232(0.9) |
| 29 | faible | vert clair | 4 | 634(46) | 24.3(1.3) | 17(3) | 25.1(1.3) | 36(6) |
| 30 | faible | vert | 5 | 903(106) | 21.8(1.4) | 6(1.3) | 21.6(1.4) | 6(1.3) |
| 31 | faible | vert foncé | 3 | 1215(92) | 17.1(0.5) | 3.4(04) | 17.1(0.5) | 3.4(0.4) |
| **34** | aucune | beige très clair | 4 | 89(6) | 10,6(0,2) | 71(14 | 16,2(0,6) | 355(34) |
| **35** | aucune | beige clair | 3 | 134(5) | 9,6(04) | 34(6) | 11,4(0,3) | 169(64) |
| **36** | aucune | beige | 3 | 174(5) | 8,6(0,2) | 22,5(2,4) | 9,6(0,1) | 143(45) |
| **37** | aucune | beige | 4 | 350(78) | 10,4(05) | 9,3(2,3) | 9,4(0,9) | 28(8) |
| **40** | aucune | semi transparente | 5 | 512(92) | 35,1(3) | 27(1) | 30,8(1,2) | 389(15) |
| **41** | aucune | beige clair | 3 | 582(84) | 28,3(2,6) | 24(2) | 24,4(1) | 66(22) |
| **42** | aucune | beige clair | 3 | 765(94) | 23,8(1) | 24(4) | 21,9(1) | 69(20) |
| **46** | aucune | beige clair | 4 | 403(38) | 26.6(7) | 32(3) | 30.3(0.8) | 160(22) |
| **47** | aucune | beige clair | 4 | 555(59) | 21.8(1) | 19(3) | 22.8(1.1) | 66(16) |
| **48** | aucune | beige clair | 4 | 836(96) | 20.7(0.9) | 7.5(1.4) | 20.5(1.1) | 15(3) |
| **49** | aucune | beige clair | 3 | 1308(37) | 19.2(07) | 3.5(0.2) | 19.2(0.6) | 3.5(0.2) |

Les produits extrudés obtenus avec les compositions bioplastiques 34-37, 40-43 ,46-49 contenant des poudres d'algues selon l'invention (exemple 3) ne dégagent aucune odeur gênante, nauséabonde ou incommodante.

Les compositions bioplastiques 16-19, 22-25, 28-32, contenant des poudres d'algues selon l'exemple 2, qui ont été préparées selon un procédé identique à celui de l'exemple 3, mais sans étape de réduction du taux de protéines, présentent une faible odeur.

Les produits extrudés obtenus avec des compositions bioplastiques élaborées à partir de la poudre d'amidon natif (compositions 1-3) et celles élaborées à partir d'algues simplement réduites en poudre de l'exemple 1 (formules 4-7, 8-11, 12-15), sans aucun traitement, présentent respectivement une odeur de pain et une forte odeur algues.

Le procédé selon l'invention est donc efficace pour supprimer les phénomènes d'odeur.

Concernant la couleur, les produits extrudés obtenus à partir d'algues simplement réduites en poudre de l'exemple 1 (formules 4-7, 8-11, 12-15), présentent des couleurs brunes sombres. De telles couleurs ne les rendent pas utilisables dans la fabrication de la majorité des produits plastiques.

Les produits extrudés obtenus avec les compositions bioplastiques 16-19 et 22-25, 28-31 contenant des poudres d'algues selon l'exemple 2, présentent des couleurs beiges claires à foncées ou vertes claires à foncées.

Seules les compositions 22 et 40 ont permis d'obtenir une couleur semi transparente. Dans ces formules, le taux d'algues est plus faible que dans les compositions 23-25 et 41-43. Le polymère est le CoPP.

Dans la formule 34 qui présente la même quantité d'algues, la couleur est beige très clair mais moins transparente, le polymère est le PBAT. Les compositions bioplastiques 34-37 et 40-43 donneront des produits plastiques dont les couleurs ne seront pas faciles à prévoir. L'ajout d'un polymère tel que le CoPP est recommandé.

Les produits extrudés obtenus avec les compositions bioplastiques 34-37 et 40-43, contenant des poudres d'algues selon l'exemple 3, présentent des couleurs beiges claires ou sont semi-transparentes. La formule 37 contenant 55 % en poids de poudre d'algues selon l'invention a permis d'obtenir un produit extrudé beige, ce qui est acceptable. En effet, lors de la formation de films, les produits sont étirés et la couleur finale rendue dépendra de l'épaisseur du film.

Ici encore, on remarque que le polymère CoPP est efficace pour accentuer la transparence.

Parmi les formulations utilisant le PBAT comme polymère, on notera que les compositions 34-36, élaborées à partir de poudres d'algues à teneur réduite en protéines, possèdent des modules d'élasticité inférieurs aux modules des autres compositions et des déformations à la rupture beaucoup plus élevées qu'avec les poudres d'algues non déprotéinées (16-18). Les matières sont donc plus souples avec une meilleure déformation.

Dans les formulations utilisant le PBS comme polymère, on notera que les compositions 47-48 avec poudre d'algue à teneur réduite en protéines possèdent des modules d'élasticité inférieurs et des déformations à la rupture beaucoup plus élevées qu'avec les poudres d'algues non déprotéinées (28-30 ), les matières sont donc plus souples avec une meilleure déformation.

Par ailleurs, les matières avec PBS et avec les mêmes taux d'algues sont cependant beaucoup plus rigides et moins déformables que les formulations en PBAT.

Dans les formulations utilisant le CoPP , on notera que les compositions ( 40-42) avec poudre d'algue à teneur réduite en protéines possèdent des modules d'élasticité et des déformations à la rupture équivalents que les poudres d'algues non déprotéinées (22-25). Les matières incorporant du CoPP sont cependant beaucoup plus rigides et moins déformables que les formulations incorporant du PBAT.

### Exemple 6 : Compositions bioplastiques formulées à partir de poudres d'algues obtenues par un procédé selon l'invention utilisant des agents anti-UV, antioxydants, anti-odeurs.

Des compositions de bioplastiques ont été formulées par mélange des poudres d'algues des exemples 2 et 3, du PBAT et des agents anti-UV, antioxydants et anti-odeurs. Au total quatre compositions ont été formulées contenant 40% de poudre d'algues.

Le Tableau 5 ci-dessous indique les compositions des mélanges réalisés :

**Tableau 5**

| Polymère | *Algues* | *Algues* | | | | | | | |
|---|---|---|---|---|---|---|---|---|---|
| | Ex. 2 | Ex. 3 | Diglycérine | PBAT | coPP | PBS | irgafos 168 | irganox 1076 | 96522 |
| PBAT | | | | | | | | | |
| 62 | 40% | | 10% | 50% | | | 0,20% | 0,20% | |
| 63 | 40% | | 10% | 50% | | | 0,20% | 0,20% | 0,40% |
| PBAT | | | | | | | | | |
| 64 | | 40% | 10% | 50% | | | 0,20% | 0,20% | |
| 65 | | 40% | 10% | 50% | | | 0,20% | 0,20% | 0,40% |

Les conditions d'extrusion ont été de 120, 140, 170°c - 50 rpm
Et les conditions d'injection ont été de 170°c - 8 bars.

Les compositions 64 et 65 sont moins colorées et sans odeurs comparées à la formule 62. La formule 63 est plus colorée mais l'odeur est améliorée par rapport à la formule 62.

L'ajout d'antioxydant et anti UV améliore la couleur par rapport aux compositions de l'exemple 4.

Les propriétés mécaniques obtenues sont les suivantes (Tableau 6)

**Tableau 6 :**

| Composition | Module Mpa | Contrainte seuil | Déformation seuil | Contrainte rupture MPA | Déformation rupture % |
|---|---|---|---|---|---|
| 62 | 150(6) | 10.1(0.7) | 21.7(0.5) | 11(0.8) | 70(26) |
| 63 | 196(5) | 10.9(1.5) | 15(5.5) | 11.7(0.7) | 40(7) |
| 64 | 204(15) | 11.3(0.9) | 18(0.6) | 11.2(1.1) | 40(6) |
| 65 | 232(22) | 12(05) | 17.2(0.4) | 11.3(0.5) | 39(3) |

### Exemple 7 : compositions bioplastiques formulées à partir de poudres d'algues obtenues par un procédé selon l'invention utilisant un autre agent chélateur à l'étape de déstructuration des parois cellulaires :

Les étapes du procédé selon l'exemple 3 ont été réalisées avec à l'étape 4 les agents chélatants suivants : oxalate de sodium, carbonate de sodium et chlorure de sodium.

Les poudres d'algues ont été mélangées avec des plastifiants et des polymères. La masse molaire de chaque mélange a été mesurée. Les résultats sont les suivants:
a) avec oxalate de sodium: 570 000 g/mol,
b) avec carbonate de sodium : 600 000 g/mol,
c) avec chlorure de sodium: 474 000 g/mol,
d) avec acide citrique : 295 000 g/mol.

La masse molaire est divisée par deux avec l'acide citrique.

Des essais de stabilité thermique ont également été menés. Les 4 produits ont été conservés 3 jours à 150°C :
- avec oxalate de sodium, chlorure de sodium et carbonate de sodium : les matériaux sont brûlés avec de fortes odeurs de caramel,
- avec l'acide citrique : la couleur reste jaune claire.

L'acide citrique est donc préféré car il limite les problèmes de compatibilité avec les autres composés. En effet, le poids moléculaire a été identifié dans le contexte de la présente invention comme un élément prépondérant pour obtenir un bon mélange des polymères, algues et autres composés. Par ailleurs, il convient de privilégier un agent d'activation qui puisse aussi être un thermostabilisant, tel que l'acide citrique.

## Revendications

1. Procédé de préparation d'une poudre d'algues, notamment destinée à la fabrication d'un produit plastique, comprenant les étapes successives de :
- culture et/ou récolte d'une biomasse d'algues,
- réduction d'au moins 70 % de la quantité intrinsèque en protéines des algues, en masse par rapport à la masse de protéines de la biomasse récoltée,
- séchage,
- réduction en poudre ou granulés.

2. Procédé de préparation d'une poudre d'algues selon la revendication 1, **caractérisé en ce que** l'étape de réduction est une étape de réduction d'au moins 75 %, de la quantité intrinsèque en protéines des algues, en masse par rapport à la masse de protéines de la biomasse récoltée.

3. Procédé de préparation d'une poudre d'algues selon la revendication 1 ou la revendication 2, **caractérisé en ce que** la réduction de la quantité de protéines de intrinsèque des algues récoltées comprend :
- l'hydrolyse enzymatique des protéines intrinsèques par mélange de la biomasse algale récoltée, préférentiellement broyée, et d'une ou plusieurs protéases, puis,
- la séparation d'un hydrolysat enrichi en peptides et/ou acides aminés et d'un résidu d'algues.

4. Procédé de préparation d'une poudre d'algues selon la revendication 3, **caractérisé en ce que** l'hydrolyse enzymatique des protéines est réalisée à l'aide d'une ou plusieurs protéases, en particulier d'une ou plusieurs endopeptidases et/ou exopeptidases, d'origine bactérienne ou fongique, telles qu'une ou plusieurs enzymes obtenues à partir de *Bacillus licheniformis, Bacillus subtilis, Bacillus amyloliquefaciens* ou *Aspergillus oryzae,* préférentiellement, de *Bacillus licheniformis.*

5. Procédé de préparation d'une poudre d'algues selon l'une des revendications 1 à 4, **caractérisé en ce qu'**il comporte, de plus, une étape de :
- dépigmentation des algues avec ou sans traitement par un agent chimique,
suite à l'étape de récolte d'une biomasse d'algues et préalablement à la réduction de la quantité intrinsèque de protéines des algues récoltées.

6. Procédé de préparation d'une poudre d'algues selon l'une des revendications 1 à 5, **caractérisé en ce qu'**il comporte, de plus, directement après l'étape de réduction de la quantité de protéines intrinsèque des algues :
- une étape de déstructuration des parois cellulaires des algues, par mélange du résidu d'algues et d'un agent solubilisant des polysaccharides, tel qu'un agent chélatant ou un acide inorganique, préférentiellement choisi parmi l'acide citrique, l'acide citrique monohydraté, l'oxalate de sodium, l'acide chlorhydrique, le carbonate de sodium, plus préférentiellement l'acide citrique, chauffage du mélange à une température comprise entre 80 et 100°, préférentiellement 90°C, pendant 1 à 4h, préférentiellement 2h, puis refroidissement et concentration du résidu d'algues, étant entendu que la dite étape ne comporte aucune élimination ou séparation des composants organiques et inorganiques.

7. Procédé de préparation d'une poudre d'algues selon la revendication 6, **caractérisé en ce que** ladite étape de déstructuration des parois cellulaires des algues comporte les étapes successives de :
- obtention d'un broyat de la biomasse algale,
- mélange dudit broyat et d'eau pour obtenir une pâte,
- ajout d'un agent solubilisant, en particulier l'acide citrique, jusqu'à un pH compris entre 2,5 et 3,5, préférentiellement 3,
- chauffage du mélange à une température comprise entre 80 et 100°, préférentiellement 90°C, pendant 1 à 4h, préférentiellement 2h,
- refroidissement à une température inférieure ou égale à 45°C,
- concentration sous vide à une température comprise entre 45 et 50°C,
- ajustement du ph du résidu obtenu à un pH compris entre 7 et 8, préférentiellement 7,7 à l'aide d'une base telle que de l'hydroxyde de sodium.

8. Procédé de préparation d'une poudre d'algues selon l'une des revendications 6 à 7, **caractérisé en ce qu'**il comporte, de plus, après l'étape de réduction de la quantité de protéines intrinsèque des algues et, lorsque le procédé comprend une étape de déstructuration des parois cellulaires des algues, directement après celle-ci, une étape d'obtention d'une pâte comprenant les étapes de :
- précipitation à l'aide d'alcool,
- séparation alcool et précipité,
- séchage du précipité.

9. Procédé selon la revendication 8, **caractérisé en ce que** le procédé comporte, de plus, immédiatement avant l'étape d'obtention d'une pâte, une étape d'ajout d'amidon dans le résidu d'algues comprenant les étapes de :
- mélange d'une dispersion d'amidon et du résidu d'algues préalablement broyé,
- chauffage du mélange à une température comprise entre 80 et 100°C durant 1 à 3 h,
- refroidissement jusqu'à une température comprise entre 45 et 50°C.

10. Procédé selon l'une des revendications précédentes, **caractérisé en ce que** le procédé comporte une étape de culture d'algues en conditions favorisant la biosynthèse d'amidon, ladite culture comprenant l'installation en bassin d'une biomasse d'algues et la culture durant quatre à six semaines, préférentiellement quatre semaines, dans un milieu de culture d'eau de mer sans apport en fertilisants, notamment sans apports en azote.

11. Procédé selon l'une des revendications précédentes, **caractérisé en ce que** les algues sont des micro algues ou des macro algues, préférentiellement des algues vertes (chlorophycées) ou brunes (phéophycées) ((rouge (rhodophycées)), plus préférentiellement des algues du genre Ulva ou de la famille des sargassaceae.

12. Poudre d'algues obtenue à l'aide d'un procédé selon l'une des revendications 1 à 11.

13. Poudre d'algues selon la revendication 12, **caractérisée en ce qu'**elle contient moins de 5% de protéines, en masse de protéines par rapport à la masse totale de la poudre.

14. Composition bioplastique, notamment destinée à la fabrication d'un produit plastique, comprenant une poudre d'algues obtenue à l'aide d'un procédé selon l'une des revendications 1 à 11.

15. Composition selon la revendication 14, **caractérisée en ce qu'**elle comprend au moins un autre composant tel qu'un composé plastifiant, un polymère naturel, un stabilisant, un antioxydant, un anti ultra-violet, un colorant, une charge, de l'eau ou un conservateur.

16. Composition selon la revendication 15, **caractérisée en ce qu'**elle comprend entre 10 et 60%, en poids, préférentiellement entre 15 et 55 %, en poids, d'une poudre d'algues obtenue à l'aide d'un procédé selon l'une des revendications 1 à 10 et entre 90 et 40 % d'au moins un autre composant.

17. Procédé de fabrication d'un produit plastique, **caractérisé en ce qu'**il comprend un procédé de préparation d'une poudre d'algues tel que décrit dans l'une des revendications 1 à 11.

18. Procédé de fabrication d'un produit plastique selon la revendication 17, **caractérisé en ce qu'**il comprend, de plus, une étape de préparation d'une composition selon l'une des revendications 14 à 16 puis une étape d'extrusion de ladite composition en granulés.

19. Procédé de fabrication d'un produit plastique selon la revendication 18, **caractérisé en ce qu'**il comprend une étape de mise en forme du produit par moulage par injection, extrusion soufflage ou extrusion en filière plate, de ladite composition de bioplastique.

20. Produit plastique obtenu à partir d'un procédé selon l'une des revendications 17 à 19.

21. Produit selon la revendication 20, **caractérisé en ce que** ledit produit est un film alimentaire, un film non alimentaire, un article moulé, des produits thermoformés, d'emballages tels que multi-couches ou de feuilles.

## Patentansprüche

1. Verfahren zur Herstellung eines Algenpulvers, das insbesondere zur Herstellung eines Kunststoffprodukts bestimmt ist, umfassend die aufeinanderfolgenden Schritte:
- Kultur und/oder Ernte einer Algenbiomasse,
- Verringern der intrinsischen Menge an Algenproteinen um mindestens 70 Massen-%, bezogen auf die Masse der Proteine der geernteten Biomasse,
- Trocknen,
- Zerkleinern zu Pulver oder Granulaten.

2. Verfahren zur Herstellung eines Algenpulvers nach Anspruch 1, **dadurch gekennzeichnet, dass** es sich bei dem Verringerungsschritt um einen Schritt der Verringerung der intrinsischen Menge an Algenproteinen um mindestens 75 Massen-%, bezogen auf die Masse der Proteine der geernteten Biomasse, handelt.

3. Verfahren zur Herstellung eines Algenpulvers nach Anspruch 1 oder Anspruch 2, **dadurch gekennzeichnet, dass** die Verringerung der intrinsischen Proteinmenge der geernteten Algen Folgendes umfasst:
- die enzymatische Hydrolyse der intrinsischen Proteine durch Mischen der geernteten, vorzugsweise zerkleinerten, Algenbiomasse und einer oder mehrerer Proteasen und
- die Trennung eines an Peptiden und/oder Aminosäuren angereicherten Hydrolysats und eines Algenrückstands.

4. Verfahren zur Herstellung eines Algenpulvers nach Anspruch 3, **dadurch gekennzeichnet, dass** die enzymatische Hydrolyse der Proteine mithilfe einer oder mehrerer aus Bakterien oder Pilzen stammender Proteasen, insbesondere einer oder mehrerer Endopeptidasen und/oder Exopeptidasen, wie eines oder mehrerer aus *Bacillus licheniformis, Bacillus subtilis, Bacillus amyloliquefaciens* oder *Aspergillus oryzae,* vorzugsweise aus *Bacillus licheniformis,* erhaltener Enzyme, durchgeführt wird.

5. Verfahren zur Herstellung eines Algenpulvers nach einem der Ansprüche 1 bis 4, **dadurch gekennzeichnet, dass** es außerdem einen Schritt:
- der Depigmentierung der Algen mit oder ohne Behandlung mit einem chemischen Mittel
nach dem Schritt der Ernte einer Algenbiomasse und vor der Verringerung der intrinsischen Proteinmenge der geernteten Algen beinhaltet.

6. Verfahren zur Herstellung eines Algenpulvers nach einem der Ansprüche 1 bis 5, **dadurch gekennzeichnet, dass** es außerdem direkt nach dem Schritt der Verringerung der intrinsischen Proteinmenge der Algen:
- einen Schritt der Destrukturierung der Zellwände der Algen durch Mischen des Algenrückstands und eines Polysaccharide solubilisierenden Mittels, wie eines Chelatbildners oder einer anorganischen Säure, die vorzugsweise aus Zitronensäure, Zitronensäure-Monohydrat, Natriumoxalat, Salzsäure, Natriumcarbonat, stärker bevorzugt aus Zitronensäure, ausgewählt sind, Erhitzen des Gemischs auf eine Temperatur zwischen 80 und 100 °C, vorzugsweise 90 °C, während 1 bis 4 Std., bevorzugt 2 Std., dann Abkühlen und Aufkonzentrieren des Algenrückstands beinhaltet, wobei selbstverständlich sein sollte, dass dieser Schritt keine Beseitigung oder Abtrennung organischer und anorganischer Verbindungen beinhaltet.

7. Verfahren zur Herstellung eines Algenpulvers nach Anspruch 6, **dadurch gekennzeichnet, dass** der Schritt der Destrukturierung der Zellwände der Algen die aufeinanderfolgenden Schritte umfasst:
- Erhalten eines Zerkleinerungsguts aus der Algenbiomasse,
- Mischen des Zerkleinerungsguts mit Wasser zum Erhalten einer Masse,
- Zugabe eines Solubilisierungsmittels, insbesondere Zitronensäure, bis auf einen pH-Wert zwischen 2,5 und 3,5, vorzugsweise 3,
- Erhitzen des Gemischs auf eine Temperatur zwischen 80 und 100 °C, vorzugsweise 90 °C, während 1 bis 4 Std., vorzugsweise 2 Std.,
- Abkühlen auf eine Temperatur von weniger als oder gleich 45 °C,
- Aufkonzentrieren unter Vakuum bei einer Temperatur zwischen 45 und 50 °C,
- Einstellen des pH-Werts des erhaltenen Rückstands auf einen pH-Wert zwischen 7 und 8, vorzugsweise 7,7, mithilfe einer Base, wie Natriumhydroxid.

8. Verfahren zur Herstellung eines Algenpulvers nach einem der Ansprüche 6 bis 7, **dadurch gekennzeichnet, dass** es außerdem nach dem Schritt der Verringerung der intrinsischen Proteinmenge der Algen und, wenn das Verfahren einen Schritt der Destrukturierung der Zellwände der Algen umfasst, direkt nach diesem einen Schritt der Gewinnung einer Masse beinhaltet, der die folgenden Schritte umfasst:
- Ausfällung mithilfe von Alkohol,
- Trennung von Alkohol und Niederschlag,
- Trocknen des Niederschlags.

9. Verfahren nach Anspruch 8, **dadurch gekennzeichnet, dass** das Verfahren außerdem unmittelbar vor dem Schritt der Gewinnung einer Masse einen Schritt der Zugabe von Stärke zu dem Algenrückstand beinhaltet, der die folgenden Schritte umfasst:
- Mischen einer Stärkedispersion und des zuvor zerkleinerten Algenrückstands,
- Erhitzen des Gemischs auf eine Temperatur zwischen 80 und 100 °C während 1 bis 3 Std.,
- Abkühlen bis auf eine Temperatur zwischen 45 und 50 °C.

10. Verfahren nach einem der vorhergehenden Ansprüche, **dadurch gekennzeichnet, dass** das Verfahren einen Schritt der Kultur von Algen unter Bedingungen umfasst, die die Biosynthese von Stärke begünstigen, wobei die Kultur das Einsetzen einer Algenbiomasse in ein Becken und eine Kultur während vier bis sechs Wochen, vorzugsweise vier Wochen, in einem Meerwasserkulturmedium ohne Einbringen von Düngemitteln, insbesondere ohne Einbringen von Stickstoff, umfasst.

11. Verfahren nach einem der vorhergehenden Ansprüche, **dadurch gekennzeichnet, dass** es sich bei den Algen um Mikroalgen oder Makroalgen, vorzugsweise Grünalgen (Chlorophyceen) oder Braunalgen (Phaeophyceen) ((Rotalgen (Rhodophyceen)), stärker bevorzugt Algen der Gattung Ulva oder der Familie der Sargassaceae, handelt.

12. Algenpulver, das mithilfe eines Verfahrens nach einem der Ansprüche 1 bis 11 erhalten wird.

13. Algenpulver nach Anspruch 12, **dadurch gekennzeichnet, dass** es weniger als 5 % Proteine, bezogen auf die Masse der Proteine in Bezug auf die Gesamtmasse des Pulvers, umfasst.

14. Biokunststoffzusammensetzung, die insbesondere zur Herstellung eines Kunststoffprodukts bestimmt ist, umfassend ein mithilfe eines Verfahrens nach einem der Ansprüche 1 bis 11 erhaltenes Algenpulver.

15. Zusammensetzung nach Anspruch 14, **dadurch gekennzeichnet, dass** sie mindestens eine andere Verbindung umfasst, wie eine Weichmacherverbindung, ein natürliches Polymer, einen Stabilisator, ein Antioxidans, ein Anti-UV-Mittel, einen Farbstoff, einen Füllstoff, Wasser oder ein Konservierungsmittel.

16. Zusammensetzung nach Anspruch 15, **dadurch gekennzeichnet, dass** sie zwischen 10 und 60 Gew.-%, vorzugsweise zwischen 15 und 55 Gew.-%, eines mithilfe eines Verfahrens nach einem der Ansprüche 1 bis 10 erhaltenen Algenpulvers und zwischen 90 und 40 % mindestens einer anderen Verbindung umfasst.

17. Verfahren zur Herstellung eines Kunststoffprodukts, **dadurch gekennzeichnet, dass** es ein Verfahren zur Herstellung eines Algenpulvers nach einem der Ansprüche 1 bis 11 umfasst.

18. Verfahren zur Herstellung eines Kunststoffprodukts nach Anspruch 17, **dadurch gekennzeichnet, dass** es außerdem einen Schritt der Herstellung einer Zusammensetzung nach einem der Ansprüche 14 bis 16 und einen Schritt der Extrusion der Zusammensetzung zu Granulaten umfasst.

19. Verfahren zur Herstellung eines Kunststoffprodukts nach Anspruch 18, **dadurch gekennzeichnet, dass** es einen Schritt der Formung des Produkts durch Spritzgießen, Extrusionsblasformen oder Schlitzdüsenextrusion der Biokunststoffzusammensetzung umfasst.

20. Kunststoffprodukt, das aus einem Verfahren nach einem der Ansprüche 17 bis 19 erhalten wird.

21. Produkt nach Anspruch 20, **dadurch gekennzeichnet, dass** es sich bei dem Produkt um eine Lebensmittelfolie, eine Nicht-Lebensmittelfolie, einen Formgegenstand, wärmegeformte Produkte, Verpackungen, wie mehrlagige oder Folien, handelt.

## Claims

1. Process for preparing an algal powder, especially intended for the manufacture of a plastic product, comprising the successive steps of:
- culturing and/or harvesting an algal biomass,
- reducing by at least 10% the intrinsic amount of proteins of the algae, by weight relative to the weight of proteins of the harvested biomass,
- drying,
- reducing to give powder or granules.

2. Process for preparing an algal powder according to Claim 1, **characterized in that** the reducing step is a step of reducing by at least 75% the intrinsic amount of proteins of the algae, by weight relative to the weight of proteins of the harvested biomass.

3. Process for preparing an algal powder according to Claim 1 or Claim 2, **characterized in that** the reducing of the intrinsic amount of proteins of the harvested algae comprises:
- the enzymatic hydrolysis of the intrinsic proteins by mixing the harvested algal biomass, preferentially ground, and one or more proteases, then
- the separation of a hydrolysate enriched in peptides and/or amino acids and an algal residue.

4. Process for preparing an algal powder according to Claim 3, **characterized in that** the enzymatic hydrolysis of the proteins is carried out by means of one or more proteases, in particular one or more endopeptidases and/or exopeptidases, of bacterial or fungal origin, such as one or more enzymes obtained from *Bacillus licheniformis, Bacillus subtilis, Bacillus amyloliquefaciens* or *Aspergillus oryzae,* preferentially from *Bacillus licheniformis.*

5. Process for preparing an algal powder according to one of Claims 1 to 4, **characterized in that** it also comprises a step of:
- depigmenting the algae with or without treatment by a chemical agent,
following the step of harvesting an algal biomass and prior to reducing the intrinsic amount of proteins of the harvested algae.

6. Process for preparing an algal powder according to one of Claims 1 to 5, **characterized in that** it also comprises, directly after the step of reducing the intrinsic amount of proteins of the algae:
- a step of destructuring the cell walls of the algae, by mixing the algal residue and a polysaccharidesolubilizing agent, such as a chelating agent or an inorganic acid, preferentially selected from citric acid, citric acid monohydrate, sodium oxalate, hydrochloric acid, sodium carbonate, more preferentially citric acid, heating the mixture to a temperature of between 80 and 100°, preferentially 90°C, for 1 to 4 h, preferentially 2 h, then cooling and concentrating the algal residue, it being understood that said step does not comprise any removal or separation of the organic and inorganic components.

7. Process for preparing an algal powder according to Claim 6, **characterized in that** said step of destructuring the cell walls of the algae comprises the successive steps of:
- obtaining a ground algal biomass material,
- mixing said ground material and water to obtain a paste,
- adding a solubilizing agent, in particular citric acid, to a pH of between 2.5 and 3.5, preferentially 3,
- heating the mixture to a temperature of between 80 and 100°, preferentially 90°C, for 1 to 4 h, preferentially 2 h,
- cooling to a temperature less than or equal to 45°C,
- concentrating under vacuum at a temperature of between 45 and 50°C,
- adjusting the pH of the residue obtained to a pH of between 7 and 8, preferentially 7.7, by means of a base such as sodium hydroxide.

8. Process for preparing an algal powder according to either of Claims 6 and 7, **characterized in that** it also comprises, after the step of reducing the intrinsic amount of proteins of the algae, and, when the process comprises a step of destructuring the cell walls of the algae, directly thereafter, a step of obtaining a paste comprising the steps of:
- precipitating by means of alcohol,
- separating alcohol and precipitate,
- drying the precipitate.

9. Process according to Claim 8, **characterized in that** the process also comprises, immediately before the step of obtaining a paste, a step of adding starch to the algal residue, comprising the steps of:
- mixing a dispersion of starch and the algal residue ground beforehand,
- heating the mixture to a temperature of between 80 and 100°C, for 1 to
3 h,
- cooling to a temperature of between 45 and 50°C.

10. Process according to one of the preceding claims, **characterized in that** the process comprises a step of culturing algae in conditions favouring the biosynthesis of starch, said culture comprising setting up an algal biomass in a tank and culturing for four to six weeks, preferentially four weeks, in a seawater culture medium without supplying fertilizers, especially without supplying nitrogen.

11. Process according to one of the preceding claims, **characterized in that** the algae are microalgae or macroalgae, preferentially green algae (Chlorophyceae) or brown algae (Pheophyceae) (red (Rhodophyceae)), more preferentially algae of the genus Ulva or the family of the Sargassaceae.

12. Algal powder obtained by means of a process according to one of Claims 1 to 11.

13. Algal powder according to Claim 12, **characterized in that** it contains less than 5% of proteins, by weight of proteins relative to the total weight of the powder.

14. Bioplastic composition, especially intended for the manufacture of a plastic product, comprising an algal powder obtained by means of a process according to one of Claims 1 to 11.

15. Composition according to Claim 14, **characterized in that** it comprises at least one other component such as a plasticizing compound, a natural polymer, a stabilizer, an antioxidant, an anti-UV agent, a dye, a filler, water or a preservative.

16. Composition according to Claim 15, **characterized in that** it comprises between 10 and 60% by weight, preferentially between 15 and 55% by weight, of an algal powder obtained by means of a process according to one of Claims 1 to 10 and between 90 and 40% of at least one other component.

17. Process for manufacturing a plastic product, **characterized in that** it comprises a process for preparing an algal powder as described in one of Claims 1 to 11.

18. Process for manufacturing a plastic product according to Claim 17, **characterized in that** it also comprises a step of preparing a composition according to one of Claims 14 to 16 then a step of extruding said composition to give granules.

19. Process for manufacturing a plastic product according to Claim 18, **characterized in that** it comprises a step of forming the product by injection moulding, extrusion blow moulding, or sheet die extrusion, of said bioplastic composition.

20. Plastic product obtained from a process according to one of Claims 17 to 19.

21. Product according to Claim 20, **characterized in that** said product is a food film, a non-food film, a moulded item, thermoformed products or packaging such as multilayers or sheets.
